# DEMANDE DE BREVET EUROPEEN

(11) **EP 1 084 699 A1**
(43) Date de publication de la demande: **21.03.2001**
(21) Numéro de dépôt: 00402378.4
(22) Date de dépôt: 28.08.2000
(51) Int. Cl.: A61K 7/11, A61K 7/06

(54) **Dispositif aérosol contenant une composition capillaire comprenant au moins un copolymère silicone/acrylate**

(30) Priorité: 16.09.1999 FR 9911595
(71) Demandeur: L'OREAL, 75008 Paris (FR)
(72) Inventeur: Dupuis, Christine, 75018 Paris (FR)
(74) Mandataire: Bourdeau, Françoise

(57) **Abrégé**

L'invention a pour objet un dispositif aérosol contenant une composition cosmétique capillaire comprenant au moins un copolymère silicone/acrylate particulier. Elle vise également un procédé cosmétique capillaire, en particulier un procédé de fixation et/ou de maintien de la coiffure mettant en oeuvre ce dispositif aérosol.

## Description

L'invention a pour objet un dispositif aérosol contenant une composition cosmétique capillaire comprenant au moins un copolymère silicone/acrylate particulier. Elle vise également un procédé cosmétique capillaire, en particulier un procédé de fixation et/ou de maintien de la coiffure mettant en oeuvre ce dispositif aérosol.

Parmi les produits capillaires pour la mise en forme et/ou le maintien de la coiffure les plus répandus sur le marché de la cosmétique, on peut citer les compositions à pulvériser essentiellement constituées d'une solution le plus souvent alcoolique ou aqueuse et d'un ou plusieurs matériaux, généralement des résines polymères, dont la fonction est de former des soudures entre les cheveux, appelés encore matériaux fixants, en mélange avec divers adjuvants cosmétiques. Cette solution est généralement conditionnée soit dans un récipient aérosol approprié mis sous pression à l'aide d'un propulseur, soit dans un flacon pompe.

Les matériaux fixants sont généralement des polymères fixants, c'est à dire des polymères filmogènes solubles dans l'eau et dans l'alcool, tels que la polyvinylpyrrolidone, les copolymères de vinylpyrrolidone/acétate de vinyle, décrits notamment dans les brevets US 3 929 735 et US 3 770 683, les copolymères d'acétate de vinyle/acide crotonique, les résines acryliques anioniques ou amphotères. Ces matériaux permettent d'obtenir facilement l'effet fixant, en revanche, après brossage ou peignage, les cheveux présentent dans les conditions usuelles de laquage un aspect raidi et un toucher rêche, voire collant ainsi qu'un poudrage après séchage.

Ces inconvénients sont liés à plusieurs paramètres, parmi lesquels on peut citer la nature du ou des polymères fixants, ou encore à la nature des soudures. Pour remédier à ces inconvénients on peut donc agir sur ces deux paramètres, sans toutefois diminuer l'effet fixant recherché. Pour améliorer les propriétés cosmétiques des matériaux fixants, il a surtout été proposé d'associer différents polymères (WO 94/12148, WO 96/06592, US 5 158 762).

La Demanderesse a maintenant trouvé qu'en sélectionnant de manière appropriée le polymère fixant et le gaz propulseur, il était possible de remédier aux différents problèmes énumérés ci-dessus, et en particulier d'obtenir un spray de bonne qualité en terme de diffusion et de taille des goutelettes, des propriétés cosmétiques satisfaisantes ainsi que peu de poudrage.

L'invention a pour objet un dispositif aérosol constitué par un récipient contenant une composition aérosol constituée, d'une part par une phase liquide (ou jus) contenant au moins un matériau fixant dans un solvant approprié et d'autre part par un propulseur, et un moyen de distribution de ladite composition aérosol, caractérisé par le fait que :
(i) le matériau fixant est un copolymère silicone/acrylate obtenu par polymérisation radicalaire d'au moins un monomère éthyléniquement insaturé (a) en présence d'au moins un dérivé siliconé (b) comprenant des groupements oxyalkylénés, et
(ii) le propulseur est choisi parmi le diméthyléther, les hydrocarbures en C3 - C4, et les mélange diméthyl éther, hydrocarbures en C3 à C5 et les mélanges d'hydrocarbures en C3 - C4, et
(iii) la composition aérosol contient plus de 10 % d'eau, dans le cas où le propulseur est le diméthyléther seul.

Un autre objet de l'invention concerne un procédé cosmétique capillaire, en particulier un procédé de fixation et/ou de maintien de la coiffure mettant en oeuvre le dispositif aérosol.

Les copolymères silicone/acrylate particulièrement visés par la présente invention sont ceux décrits dans la demande internationale WO99/04750. En particulier, on préfère le copolymère commercialisé par BASF sous la dénomination Luviflex Silk. Ce polymère un copolymère greffé acrylate de tertiobutyle/ acide méthacrylique et silicone copolyol.

De préférence, le monomère (a) répond à la formule (Iₐ) : 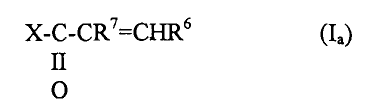 dans laquelle :
- X est choisi dans le groupe comprenant OH, OM, OR⁸, NH₂, NHR⁸, N(R⁸)₂ ;
- M est un cation choisi parmi Na⁺, K⁺, Mg⁺⁺, NH ⁴⁺ , alkylammonium, dialkylammonium, trialkylammonium, et tétraalkylammonium ;
- les radicaux R⁸ peuvent être identiques ou différents et sont choisis dans le groupe comprenant l'hydrogène, les groupements alkyls linéaires ou ramifiés en C₁ à C₄₀, les groupements alkyls en C₁ à C₄₀ mono- ou polyhydroxylés, éventuellement substitués par un ou plusieurs groupement(s) alcoxy, amino ou carboxy, les groupements polyéthers hydroxylés, les groupements N,N-diméthylaminoéthyl, 2-hydroxyéthyl, 2-méthoxyéthyl, 2-éthoxyéthyl, hydroxypropyl, méthoxypropyl et éthoxypropyl ;
- R⁷ et R⁶ sont choisis indépendamment l'un de l'autre dans le groupe comprenant l'hydrogène, les groupements alkyls linéaires ou ramifiés en C₁ à C₈, méthoxy, éthoxy, 2-hydroxyéthoxy, 2-méthoxyéthoxy et 2-éthoxyéthyl, les groupements CN, COOH et COOM.

En particulier, les monomères (a) de formule (la) sont l'acide acrylique et ses sels, esters ou amides. Les esters peuvent être dérivés d'alkyles linéaires en C₁ à C₄₀, d'alkyles ramifiés en C₃ à C₄₀ ou d'alcools carboxyliques en C₃ à C₄₀, d'alcools polyfonctionnels ayant 2 à 8 hydroxyles, tel que l'éthylène glycol, l'hexylène glycol, le glycérol et le 1,2,6-hexanetriol, d'aminoalcools ou d'éthers d'alcools tels que le méthoxyméthanol et l'éthoxyéthanol ou les polyalkylènes glycols.

Les monomères (a) de formule (la) peuvent également être choisis parmi les N,N-dialkylaminoalkyl acrylates et méthacrylates et N-dialkylaminoalkylacryl- et -méthacrylamides, le groupement amide pouvant être non substitué, N-aklyl- ou N-alkylamino-monosubstitué ou N,N-dialkylamino-disubstitué, les groupes alkyles ou alkylamino étant dérivés d'unités carboxyliques linéaires en C₁ à C₄₀ ou ramifiées en C₃ à C₄₀.

Les monomères (a) de formule (la) pouvant être utilisés peuvent aussi être des composés substitués dérivés de l'acide acrylique ou ses sels, esters ou amides. On peut citer par exemple les acides méthacrylique, éthacrylique et 3-cyanoacrylique.

D'autres monomères (a) convenant particulièrement bien sont les esters de vinyle et d'allyle en C₁ à C₄₀, les acides carboxyliques, linéaires en C₃ à C₄₀, ou carboxycycliques en C₃ à C₄₀, les halides de vinyle ou d'allyle, les vinyllactames, de préférence la vinylpyrrolidone et le vinylcaprolactame, et les composés hétérocycles substitués par des groupement vinyles ou allylse, de préférence la vinylpyridine, la vinyloxazoline et l'allylpyridine, les N-vinylimidazoles, les diallylamines, le chlorure de vinylidène, les composés insaturés carbonés, comme le styrène ou l'isoprène, les dérivés quaternisés par l'épichlorhydrine de l'acide acrylique ou méthacrylique.

Comme autre monomère (a), on peut enfin citer les N-vinylimidazoles, les diallylamines, le chlorure de vinylidène, les composés insaturés carbonés comme le styrène et l'isoprène, les dérivés quaternisés par l'épichlorohydrine de l'acide acrylique ou méthacrylique.

On préfère tout particulièrement, comme monomère (a) l'acide acrylique, méthacrylique et éthylacrylique; le méthyl, éthyl, propyl, n-butyl, isobutyl, t-butyl, 2-éthylhexyl et décyl acrylate ; le méthyl, éthyl, propyl, n-butyl, isobutyl, t-butyl, 2-éthylhexyl et décyl méthacrylate ; le méthyl, éthyl, propyl, n-butyl, isobutyl, t-butyl, 2-éthylhexyl et décyl éthacrylate ; le 2,3-dihydroxypropyl acrylate ; le 2,3-dihydroxypropyl méthacrylate ; le 2-dihydroxyéthyl acrylate ; l'hydroxypropyl acrylate ; le 2-hydroxyéthyl méthacrylate ; le 2-hydroxyéthyl éthacrylate ; le 2-méthoxyéthyl acrylate ; le 2-éthoxyéthyl méthacrylate ; le 2-éthoxyéthyl éthacrylate; l'hydroxypropyl méthacrylate ; le glycéryl monoacrylate; le glycéryl monométhacrylate; les polyalkylènes glycols( méth-) acrylate, les acides sulfoniques insaturés, l'acrylamide, la méthacrylamide, l'éthacrylamide, la N,N-diméthylacrylamide, la N-éthylacrylamide, la N-éthylméthacrylamide, 1-vinylimidazole, N,N-diméthylaminoéthyl(méth-)acrylate, l'acide maléique, l'acide fumarique, l'anhydride maléique et ses monoesters, l'acide crotonique, l'acide itaconique, les éthers de vinyle, le vinylformamide, la vinylamine, la vinylpyridine, la vinylimidazole, le vinylfurane, le styrène, le sulfonate de styrène, l'alcool allylique et leurs mélanges.

Le monomère (a) peut contenir également des atomes de silicium, de fluor ou encore des groupements thio. Les monomères (a) peuvent être neutralisés s'ils contiennent des groupement acides, et ceci avant ou après la polymérisation, totalement ou partiellement, de façon à ajuster la solubilité ou le degré de dispersion dans l'eau au niveau désiré. En tant qu'agent servant pour la neutralisation, on peut utiliser les bases minérales, telles que le carbonate de sodium, les bases oraganiques telles que les aminoalcools, comme les alcanolamines telle que la méthanolamine, le 2-amino-2-méthyl-1-propanol, la triéthanolamine, les diamines comme la lysine.

Les monomères (a) peuvent être aussi quaternisés lorsqu'ils possèdent un atome d'azote basique. S'ils possèdent au moins deux doubles liaisons éthyléniques, les monomères (a) peuvent être partiellement réticulés.

Les dérivés siliconés (b) sont notamment les composés connus, sous la dénomination INCI par diméthicone copolyols ou les tensioactifs siliconés. On peut citer ceux commercialisés sous la marque Abil ® par Goldschmidt, Alkasil ® par Rhône-Poulenc, silicone Polyol Copolymer ® par Genesee, Besil ® par Wacker, Silwet ® par OSI ou Dow Corning 190® par Dow Corning.

Parmi les dérivés siliconés (b), on préfère ceux qui présentent la formule I ci-après : 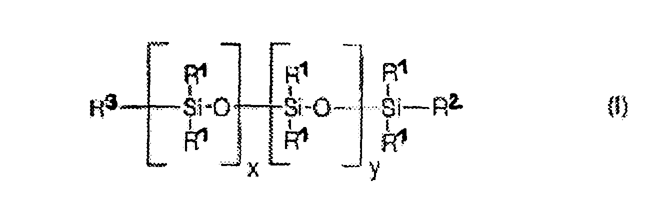 dans laquelle formule (I):
- R² est choisi parmi CH₃ ou le groupement 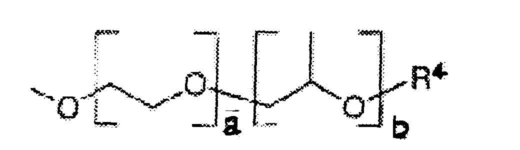
- R³ est choisi parmi CH₃ ou le groupement R²,
- R⁴ est choisi parmi l'hydrogène, CH₃, ou les groupements 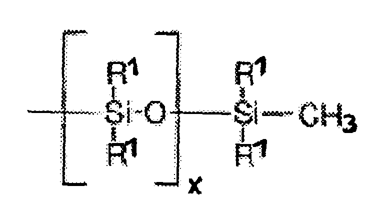 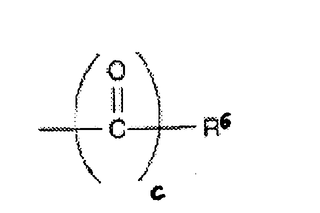
- R⁶ est un groupement organique en C₁ à C₄₀ pouvant contenir des groupements amino, carboxyles ou sulfonates, et si c=0, R₆ est l'anion d'un acide inorganique ;
- les radicaux R¹ peuvent être identiques ou différents et sont choisis parmi les hydrocarbures aliphatiques en C₁ à C₂₀, les hydrocarbures aliphatiques ou cycloaliphatiques en C₃ à C₂₀, et les groupements R⁵ répondant à la formule ci-après : 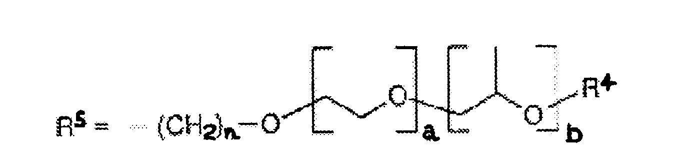
- n est un nombre entier compris entre 1 et 6,
- x et y sont des nombres entiers choisis de telle sorte que le poids moléculaire du polysiloxane soit compris entre 300 et 30 000,
   - a et b sont des nombres entiers compris entre 0 et 50, et
- c est 0 ou 1.

De préférence, R¹ est choisi parmi les groupements méthyl, éthyl, propyl, butyl, isobutyl, pentyl, isopentyl, hexyl, octyl, décyl, dodécyl and octadécyl, les radicaux cycloaliphatiques, en particulier les groupements cyclohexyls, les groupements aromatiques, en particulier les groupements phényls ou naphthyls, les mélanges de radicaux aromatiques et aliphatiques, tels que le benzyl et le phenyléthyl, et aussi le tolyl and le xylyl.

On préfère les radicaux R⁴ ayant pour formule -(CO)c-R⁶, R⁶ étant un alkyl, cycloalkyl or aryl ayant 1 à 40 atomes de carbone qui peut contenir des groupements supplémentaires tels que NH₂, COOH et/ou SO₃H.

On préfère les radicaux R⁶ pour lesquels c=0 qui sont des phosphates ou des sulfates.

Les dérivés siliconés (b) particulièrement préférés sont ceux présentant la formule générale suivante :

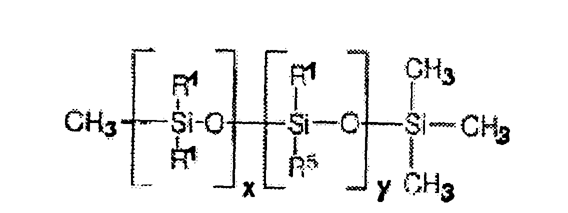

La proportion relative de dérivé (b) dans le copolymère est généralement de 0,1 à 50, et de préférence de 1 à 20 % en poids.

On préfère les copolymères silicone/acrylate solubles dans l'eau ou ceux dont la dispersibilité dans l'eau est telle que dans un mélange eau/éthanol dosé à 50 :50 en volume, ils sont solubles dans une proportion supérieure à 0,1 g/l, et de préférence supérieure à 0,2 g/l. 7. Avantageusement, la composition selon l'invention comprend, en pourcentage relatif en poids, de 0,1 à 20 % de copolymère silicone/acrylate, et plus préférentiellement de 0,5 à 10 % de ce copolymère. Le dispositif aérosol comprend avantageusement de 10 à 90 % de propulseur, et de préférence de 20 à 70 %.

Selon un premier mode de réalisation avantageux du dispositif conforme à l'invention, le propulseur est le diméthyl éther, et celui-ci est avantageusement présent, dans le dispositif aérosol, à une concentration allant de 30 à 60 %.

Selon un deuxième mode de réalisation avantageux du dispositif conforme à l'invention, le propulseur est un hydrocarbure en C3 à C4, et celui-ci est avantageusement présent, dans le dispositif aérosol, à une concentration allant de 10 à 70 %. De préférence, on choisit l'hydrocarbure parmi le n-butane, le propane, l'isobutane, les hydrocarbures halogénés et leurs mélanges.

Selon un troisième mode de réalisation avantageux du dispositif conforme à l'invention, le propulseur est un mélange de diméthyl éther et d'hydrocarbure(s) en C3 à C5, et le rapport diméthyl éther:hydrocarbure(s) est avantageusement compris entre 10 : 90 et 90 : 10.

Le milieu cosmétiquement acceptable est de préférence constitué par de l'eau ou un ou plusieurs solvants cosmétiquement acceptables ou des mélanges eau-solvants. Ces solvants sont, de préférence, des alcools en C1-C4, l'éthanol étant particulièrement préféré.

De manière avantageuse, le solvant approprié contient au moins 50 % en volume d'alcool, de préférence au moins 70 % en volume d'alcool.

La composition de l'invention peut également contenir au moins un additif choisi parmi les tensioactifs anioniques, cationiques, non ioniques ou amphotères, les parfums, les filtres, les conservateurs, les protéines, les vitamines, les polymères autres que ceux de l'invention, et en particulier les polymères fixants anioniques, amphotères ou non ioniques, les huiles végétales, minérales ou synthétiques et tout autre additif classiquement utilisé dans les compositions cosmétiques.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions conformes à l'invention sont particulièrement adaptés pour des cheveux secs ou humides, en tant que produits de coiffage.

Les compositions conformes à l'inventions peuvent être appliquées sur la peau, les ongles, les lèvres, les cheveux, les sourcils et les cils.

L'invention va être plus complètement illustrée à l'aide des exemples non limitatifs suivants.

Tous les pourcentages sont des pourcentages relatifs en poids par rapport au poids total de la composition et m.a. signifie matière active.

### EXEMPLE:

### Composition A

On réalise le dispositif aérosol suivant conforme à l'invention.

| Jus : | |
|---|---|
| Luviflex Silk * | 6 % |
| AMP | qs 100 % neutralisation |
| Ethanol | qs 100 g |

| Schéma de pressurisation : | |
|---|---|
| Jus | 37 % |
| DME | 43 % |
| Pentane | 20 % |

| | |
|---|---|
| * copolymère silicone acrylate proposé par BASF (t-butylacrylate/acide méthacrylique/PDMS polyéther) | |

### Composition B

| Jus : | |
|---|---|
| Luviflex Silk * | 4 % |
| Amphomer LV 71 | 2 % |
| AMP | qs 100 % neutralisation |
| Eau | 16% |
| Ethanol | 78 % |

| Schéma de pressurisation : | |
|---|---|
| Jus | 65 % |
| DME | 35 % |

| | |
|---|---|
| * copolymère silicone acrylate proposé par BASF (t-butylacrylate/acide méthacrylique/PDMS polyéther) | |

Ces deux compositions sont pulvérisées individuellement sur des cheveux eurochâtains. Les cheveux ont de bonnes propriétés cosmétiques dans les deux cas, après séchage.

## Revendications

1. Dispositif aérosol constitué par un récipient contenant une composition aérosol constituée, d'une part par une phase liquide (ou jus) contenant au moins un matériau fixant dans un solvant approprié et d'autre part par un propulseur, et un moyen de distribution de ladite composition aérosol, caractérisé par le fait que :
(i) le matériau fixant est un copolymère silicone/acrylate obtenu par polymérisation radicalaire d'au moins un monomère éthyléniquement insaturé (a) en présence d'au moins un dérivé siliconé (b) comprenant des groupements oxyalkylénés, et
(ii) le propulseur est choisi parmi le diméthyléther, les hydrocarbures en C3 - C4, et les mélange diméthyl éther, hydrocarbures en C3 à C5 et les mélanges d'hydrocarbures en C3 - C4, et
(iii) la composition aérosol contient plus de 10 % d'eau, dans le cas où le propulseur est le diméthyléther seul.

2. Dispositif selon la revendication 1, caractérisé par le fait que le monomère (a) répond à la formule (Iₐ) : 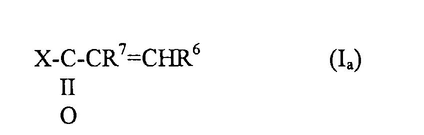 dans laquelle :
- X est choisi dans le groupe comprenant OH, OM, OR⁸, NH₂, NHR⁸, N(R⁸)2;
- M est un cation choisi parmi Na⁺, K⁺, Mg⁺⁺, NH ⁴⁺ , alkylammonium, dialkylammonium, trialkylammonium, et tétraalkylammonium ;
- les radicaux R⁸ peuvent être identiques ou différents et sont choisis dans le groupe comprenant l'hydrogène, les groupements alkyls linéaires ou ramifiés en C₁ à C₄₀, les groupements alkyls en C₁ à C₄₀ mono- ou polyhydroxylés, éventuellement substitués par un ou plusieurs groupement(s) alcoxy, amino ou carboxy, les groupements polyéthers hydroxylés, leles groupements N,N-diméthylaminoéthyl, 2-hydroxyéthyl, le 2-méthoxyéthyl, 2-éthoxyéthyl, hydroxypropyl, méthoxypropyl et éthoxypropyl ;
- R⁷ et R⁶ sont choisis indépendamment l'un de l'autre dans le groupe comprenant l'hydrogène, les groupements alkyls linéaires ou ramifiés en C₁ à C₈, méthoxy, éthoxy, 2-hydroxyéthoxy, 2-méthoxyéthoxy et 2-éthoxyéthyl, les groupements CN, COOH et COOM.

3. Dispositif selon la revendication 1, caractérisée par le fait que le dérivé siliconé (b) répond à la formule I ci-après : 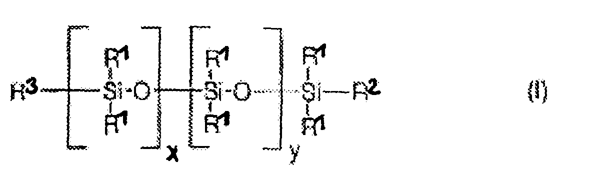 dans laquelle formule (I):
- R² est choisi parmi CH₃ ou le groupement 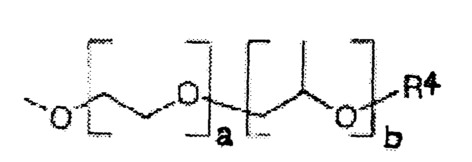
- R³ est choisi parmi CH₃ ou le groupement R²,
- R⁴ est choisi parmi l'hydrogène, CH₃, ou les groupements 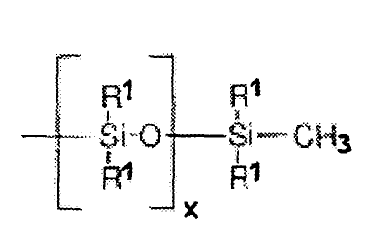 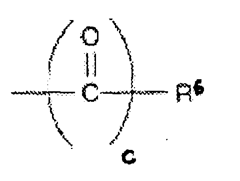
- R⁶ est un groupement organique en C₁ à C₄₀ pouvant contenir des groupements amino, carboxyles ou sulfonates, et si c=0, R₆ est l'anion d'un acide inorganique ;
- les radicaux R¹ peuvent être identiques ou différents et sont choisis parmi les hydrocarbures aliphatiques en C₁ à C₂₀, les hydrocarbures aliphatiques ou cycloaliphatiques en C₃ à C₂₀, et les groupements R⁵ répondant à la formule ci-après : 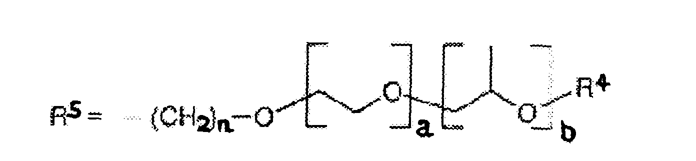
- n est un nombre entier compris entre 1 et 6,
- x et y sont des nombres entiers choisis de telle sorte que le poids moléculaire du polysiloxane soit compris entre 300 et 30 000,
- a et b sont des nombres entiers compris entre 0 et 50, et
- c est 0 ou 1.

4. Dispositif selon la revendication 1, caractérisé par le fait que le monomère (a) est choisi parmi les esters de vinyle et d'allyle en C₁ à C₄₀, les acides carboxyliques, linéaires en C₃ à C₄₀, ou carboxycycliques en C₃ à C₄₀, les halides de vinyle ou d'allyle, les vinyllactames, de préférence la vinylpyrrolidone et le vinylcaprolactame, et les composés hétérocycles substitués par des groupement vinyles ou allyles, de préférence la vinylpyridine, la vinyloxazoline et l'allylpyridine, les N-vinylimidazoles, les diallylamines, le chlorure de vinylidène, les composés insaturés carbonés, comme le styrène ou l'isoprène, les dérivés quaternisés par l'épichlorhydrine de l'acide acrylique ou méthacrylique.

5. Dispositif selon la revendication 1, caractérisé par le fait que le monomère (a) est choisi dans le groupe comprenant l'acide acrylique, méthacrylique et éthylacrylique ; le méthyl, éthyl, propyl, n-butyl, isobutyl, t-butyl, 2-éthylhexyl et décyl acrylate ; le méthyl, éthyl, propyl, n-butyl, isobutyl, t-butyl, 2-éthylhexyl et décyl méthacrylate ; le méthyl, éthyl, propyl, n-butyl, isobutyl, t-butyl, 2-éthylhexyl et décyl éthacrylate ; le 2,3-dihydroxypropyl acrylate ; le 2,3-dihydroxypropyl méthacrylate; le 2-dihydroxyéthyl acrylate ; l'hydroxypropyl acrylate ; le 2-hydroxyéthyl méthacrylate; le 2-hydroxyéthyl éthacrylate ; le 2-méthoxyéthyl acrylate ; le 2-éthoxyéthyl méthacrylate ; le 2-éthoxyéthyl éthacrylate ; l'hydroxypropyl méthacrylate ; le glycéryl monoacrylate ; le glycéryl monométhacrylate ; les polyalkylènes glycols( méth-) acrylate, les acides sulfoniques insaturés, l'acrylamide, la méthacrylamide, l'éthacrylamide, la N,N-diméthylacrylamide, la N-éthylacrylamide, la N-éthylméthacrylamide, 1-vinylimidazole, N,N-diméthylaminoéthyl(méth-)acrylate, l'acide maléique, l'acide fumarique, l'anhydride maléique et ses monoesters, l'acide crotonique, l'acide itaconique, les éthers de vinyle, le vinylformamide, la vinylamine, la vinylpyridine, la vinylimidazole, le vinylfurane, le styrène, le sulfonate de styrène, l'alcool allylique et leurs mélanges.

6. Dispositif selon l'une quelconque des revendications précédentes, caractérisé par le fait que les dérivés siliconés (b) sont choisis parmi les diméthicone copolyols ou les tensioactifs siliconés.

7. Dispositif selon l'une quelconque des revendications précédentes, caractérisé par le fait que la composition comprend, en pourcentage relatif en poids, de 0,1 à 20 % de copolymère silicone/acrylate, et plus préférentiellement de 0,5 à 10 % de ce copolymère.

8. Dispositif selon l'une quelconque des revendications précédentes, caractérisé par le fait que le propulseur est le diméthyl éther, et celui-ci est avantageusement présent, dans le dispositif aérosol, à une concentration allant de 30 à 60 %.

9. Dispositif selon l'une quelconque des revendications 1 à 7, caractérisé par le fait que le propulseur est un hydrocarbure en C3 à C4, et celui-ci est présent, dans le dispositif aérosol, à une concentration allant de 10 à 70 %.

10. Dispositif selon l'une quelconque des revendications 1 à 7, caractérisé par le fait que le propulseur est un mélange de diméthyl éther et d'hydrocarbure(s) en C3 à C5, et le rapport diméthyl éther:hydrocarbure(s) est compris entre 10 : 90 et 90 : 10.

11. Dispositif selon l'une quelconque des revendications précédentes, caractérisée par le fait que la composition contient en outre au moins un additif choisi parmi les tensioactifs anioniques, cationiques, non ioniques ou amphotères, les parfums, les filtres, les conservateurs, les protéines, les vitamines, les polymères autres que ceux de l'invention, et en particulier les polymères fixants anioniques, amphotères ou non ioniques, les huiles végétales, minérales ou synthétiques.

12. Procédé de maintien ou de mise en forme de la coiffure, caractérisé par le fait qu'il comprend la mise en oeuvre d'un dispositif conforme à l'une quelconque des revendications 1 à 11.

13. Utilisation d'un dispositif selon l'une quelconque des revendications 1 à 11, pour la fabrication d'un produit cosmétique, en particulier capillaire.

14. Utilisation d'une composition selon l'une quelconque des revendications 1 à 11 pour la peau, les ongles, les lèvres, les cheveux, les sourcils et les cils.
